**Europäisches Patentamt**

**European Patent Office**

**Office européen des brevets**

(19)

(11) Veröffentlichungsnummer: **0 335 222 B1**

## (12) EUROPÄISCHE PATENTSCHRIFT

(45) Veröffentlichungstag der Patentschrift: **09.12.92**

(21) Anmeldenummer: **89104992.6**

(22) Anmeldetag: **21.03.89**

(51) Int. Cl.⁵: **B01J 23/74**, C07C 5/10, C07C 5/03, C07C 5/05, C07C 209/40, C07C 209/42, C07C 209/48, C07C 209/50, B01J 37/03

(54) **Verfahren zur Herstellung Nickel, Aluminiumoxyd und Zirkondioxyd enthaltender Katalysatorzusammensetzungen.**

(30) Priorität: **31.03.88 DE 3811038**

(43) Veröffentlichungstag der Anmeldung:
**04.10.89 Patentblatt 89/40**

(45) Bekanntmachung des Hinweises auf die Patenterteilung:
**09.12.92 Patentblatt 92/50**

(84) Benannte Vertragsstaaten:
**AT BE CH DE ES FR GB IT LI NL SE**

(56) Entgegenhaltungen:
**EP-A- 0 097 047          GB-A- 2 066 690**
**US-A- 2 564 331          US-A- 4 307 248**
**US-A- 4 384 985          US-A- 4 687 568**

(73) Patentinhaber: **HOECHST AKTIENGESELL-SCHAFT**
**Postfach 80 03 20**
**W-6230 Frankfurt am Main 80(DE)**

(72) Erfinder: **Horn, Gerhardt, Dr. Dipl.-Chem.**
**Bunsenstrasse 19**
**W-4200 Oberhausen 11(DE)**
Erfinder: **Frohning, Carl Dieter, Dr. Dipl.-Chem.**
**Regnitzstrasse 50**
**W-4230 Wesel(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 335 222 B1

**Beschreibung**

Die vorliegende Erfindung betrifft ein Verfahren zur Herstellung einer Nickel, Aluminiumoxid und Zirkondioxid enthaltenden Katalysatorzusammensetzung durch gleichzeitige Fällung von Nickel, Aluminium und Zirkon aus einer Mischsalzlösung mittels einer wäßrigen Lösung einer basischen Verbindung als Fällungsmittel, Abscheiden auf einem Trägermaterial, Filtration, Trocknung und Reduktion, sowie eine spezifische Katalysatorzusammensetzung und deren Verwendung.

In der SU-PS 28 31 85 sind Katalysatoren, die ausschließlich Nickel, Aluminiumoxid und Zirkondioxid enthalten, beschrieben. Sie werden durch Fällung von Ni und Aluminiumoxid auf Zirkondioxid als Träger hergestellt.

Die US-PS 38 76 557 beschreibt ein Verfahren zur Herstellung von Katalysatoren durch gleichzeitige Fällung von Metallen, wobei die Metalle unter Einhaltung eines pH-Wertes von 1,0 bis 3,0 in Form ihrer unlöslichen Oxalate gefällt werden. Es werden Ni, Co, Fe, Cu, Zn, Zr, Al, Ba, Ca, Sr und Mg als Metalle genannt.

In der US-PS 2 564 331 ist die Herstellung von aktiven Nickel-Zirkondioxid-Katalysatoren beschrieben. Zu einer im großen Überschuß vorliegenden, wäßrigen Natriumcarbonatlösung wird eine wäßrige Lösung von Zirkonsulfat gegeben, wobei zunächst eine basische Zirkonverbindung ausfällt, die sich jedoch infolge des großen Überschusses an Carbonationen wieder auflöst. Dieser Lösung wird bei einer Temperatur von etwa 74 °C eine wäßrige Nickelsulfat-Lösung zugesetzt. Es bildet sich ein aus Nickel-Zirkoncarbonat bestehender Mischniederschlag, der mittels Waschen, Trocknen, Kalcinieren und Reduzieren weiter verarbeitet wird.

Die DE-AS 12 57 753 beschreibt ein Verfahren zur Herstellung eines durch Zirkondioxid aktivierten Nickelkatalysators durch Fällung der unlöslichen Carbonate. Man geht von einer wäßrigen Mischsalzlösung von Ammoniumzirkonylcarbonat und Nickelammincarbonat aus und dampft aus dieser Lösung soviel Ammoniak und Kohlendioxid ab, daß ein Gemisch basischer Carbonate bei etwa 82 °C und darüber ausfällt. Nach Filtration wird getrocknet, kalziniert und reduziert.

Bei der Herstellung von Katalysatorzusammensetzungen, die aus zwei oder drei Komponenten bestehen, mittels Fällung, ist darauf zu achten, daß die Fällung zu einem möglichst homogenen Gemisch der einzelnen Komponenten führt. Dies ist mit den vorstehend genannten Verfahren nur unzureichend sichergestellt.

Gemäß der Arbeitsweise der US-PS 25 64 331 verwendet man einen hohen Carbonatüberschuß. Dies hat zur Folge, daß zuerst reines Nickelcarbonat ausfällt und die eigentliche Mischfällung lediglich in einem engen Bereich stattfindet, während gegen Ende der Fällung ausschließlich Zirkoncarbonat ausgeschieden wird.

Folgt man den in der US-PS 38 67 557 und DE-AS 12 57 753 beschriebenen Verfahren, so bewirkt man durch Zugabe einer wäßrigen Oxalsäurelösung zu einer Metallmischsalzlösung einerseits und durch Abdampfen von Ammoniak und Kohlendioxid andererseits eine allmähliche Verschiebung des pH-Wertes während der Fällung.

Da die Löslichkeit der einzelnen Komponenten stark vom jeweils vorherrschenden pH-Wert abhängt, scheidet sich stets ein dem pH-Wert entsprechendes Fällungsgemisch ab. Da das Fällungsgemisch in Abhängigkeit vom pH-Wert seine Zusammensetzung ändert, erhält man infolge der pH-Wertänderung unterschiedlich zusammengesetzte, nicht homogene Fällungsgemische.

Da diese Schwierigkeiten bereits bei der Fällung von nur zwei Komponenten - beispielsweise Nickel- und Zirkonverbindungen - auftreten, ist davon auszugehen, daß eine Fällung von aus drei Metallverbindungen bestehenden, homogenen Copräzipitaten besonderen Anforderungen genügen muß.

Darüber hinaus soll die Katalysatorzusammensetzung bessere Eigenschaften als bislang bekannte Katalysatoren aufweisen.

Die Aufgabe wird gelöst durch ein Verfahren zur Herstellung einer Nickel, Aluminiumoxid und Zirkondioxid enthaltenden Katalysatorzusammensetzung durch Fällung, Filtration, Trocknung und Reduktion.

Es ist dadurch gekennzeichnet, daß eine wäßrige Ni-Al-Zr-Mischsalzlösung mit einer wäßrigen Lösung einer basischen Verbindung als Fällungsmittel vermischt, die basische Verbindung in einem stöchiometrischen Überschuß von 5 bis 100 %, bezogen auf die zur Fällung von Ni, Al und Zr erforderlichen Menge, eingesetzt wird, Ni, Al und Zr gleichzeitig gefällt und auf einem Trägermaterial abgeschieden werden, die Fällung bei 60 bis 120 °C und pH 7 bis 10 durchgeführt wird.

Um einer unerwünschten Hydrolyse vorzubeugen und die Fällung günstig zu beeinflussen, empfiehlt es sich, der Mischsalzlösung freie Säure im Überschuß zuzufügen. Die Mischsalzlösung soll freie Säure entsprechend einem Verhältnis $H^+ : Zr^{4+}$ = (2 bis 40) : 1, insbesondere (3 bis 30) : 1, bevorzugt (4 bis 20) : 1, enthalten. Die freie Säure wird durch Titration mit NaOH bestimmt, indem man solange NaOH zusetzt, bis pH = 0,8 erreicht wird.

Als freie Säure können Salzsäure, Schwefelsäure und Salpetersäure eingesetzt werden. Besonders geeignet ist Salpetersäure.

Die Mischsalzlösung besteht aus 10 bis 100, insbesondere 20 bis 80, bevorzugt 30 bis 50 g Ni/l. Sie weist Aluminium entsprechend 1 bis 30, insbesondere 3 bis 15, bevorzugt 4 bis 10 Gew.-Teilen $Al_2O_3$ je 100 Gew.-Teile Ni auf. Ferner enthält sie Zirkon entsprechend 0,5 bis 20, insbesondere 1 bis 10, bevorzugt 1,5 bis 5 Gew.-Teilen $ZrO_2$ je 100 Gew.-Teile Ni.

Die Mischsalzlösung stellt man her, indem man wasserlösliche anorganische, organische oder komplexe Salze von Nickel, Zirkon und Aluminium in Wasser löst. Gut geeignete Salze sind die Sulfate, Chloride, Acetate, Propionate, Butyrate und Nitrate. Besonders bewährt hat es sich, Nickel, Aluminium und Zirkon in Form der Sulfate, Chloride, Acetate und Nitrate, bevorzugt in Form ihrer Nitrate einzusetzen.

Als Fällungsmittel dient eine wäßrige Lösung einer basischen Verbindung, insbesondere eine wäßrige Alkali-carbonat-, Alkalihydrogencarbonat-, Alkalihydroxid-, Ammoniumhydroxid- oder Ammoniumcarbonatlösung. Es können auch Mischungen derselben Verwendung finden. Besonders geeignet ist eine wäßrige $Na_2CO_3$ und/oder $NaHCO_3$ enthaltende Lösung. Das Fällungsmittel soll einen pH-Wert von 7,5 bis 13, insbesondere 8 bis 12, bevorzugt von 9 bis 11, aufweisen.

Das Fällungsmittel enthält 0,1 bis 4,0, insbesondere 0,6 bis 3,0, bevorzugt 1,6 bis 2,4 Äquivalente basische Verbindung/l Lösung. Recht gute Ergebnisse werden mit wäßrigen Lösungen, die 0,3 bis 1,5, insbesondere 0,8 bis 1,2 Mol Alkalicarbonat/l Lösung aufweisen, erzielt.

Um eine möglichst vollständige Fällung eines besonders homogenen Copräzipitats sicherzustellen, setzt man die basische Verbindung im Überschuß ein. Im allgemeinen beträgt der stöchiometrische Überschuß 5 bis 100 %, insbesondere 10 bis 70 % basische Verbindung. Besonders bewährt hat sich die Verwendung von 20 bis 40 % überschüssige basische Verbindung. Der Überschuß bezieht sich jeweils auf die zur vollständigen Fällung von Ni, Al und Zr erforderliche Menge basische Verbindung.

Der stöchiometrische Überschuß ist derart zu bemessen, daß einerseits zwar eine Fällung eines homogenen Coprazipitäts gewährleistet ist, andererseits aber auch eine quantitative Fällung der in der Mischsalzlösung enthaltenen drei Metalle eintritt.

Die Fällung wird herbeigeführt, indem man entweder die Mischsalzlösung und das Fällungsmittel kontinuierlich zusammenführt und vermischt oder, gemäß einer bevorzugten Variante, das Fällungsmittel vorlegt und die Mischsalzlösung in das Fällungsmittel einleitet.

Besonders einfach gestaltet sich die letztere Variante, da nur die Zugabegeschwindigkeit einer Lösung, nämlich die der Mischsalzlösung einzustellen und zu kontrollieren ist. Daher wird man in vielen Fällen dieser Arbeitsweise den Vorzug geben.

Das Trägermaterial kann mit der Mischsalzlösung und/oder mit dem Fällungsmittel in die Reaktion eingesetzt werden. Bevorzugt man diese Arbeitsweise, so suspendiert man das Trägermaterial in der Mischsalzlösung und/oder in dem Fällungsmittel.

Es hat sich als besonders vorteilhaft erwiesen, zunächst die Mischsalzlösung und das Fällungsmittel miteinander zu vermischen und anschließend das Trägermaterial zuzusetzen. Arbeitet man nach dieser Variante, so erhält man eine Katalysatorzusammensetzung, deren physikalische Eigenschaften insbesondere Festigkeit und Schüttdichte, die weitere Verarbeitung erleichtern.

Als Trägermaterial eignen sich Aktivkohle, Tonerden, Bimsstein, $\gamma$-$Al_2O_3$, $SiO_2$, Kieselgel, Kieselgur und Kieselerden. Besonders haben sich $SiO_2$, Kieselgel, Kieselgur und Kieselerde bewährt. Bevorzugt werden Kieselgur und $SiO_2$ in Form gefällter Kieselsäure eingesetzt.

Das erfindungsgemäße Verfahren bietet die Möglichkeit, Katalysatorzusammensetzungen auf feinteiligem Trägermaterial herzustellen. Das Trägermaterial besitzt eine Korngröße von 0,1 bis 200, insbesondere 0,5 bis 50, bevorzugt 1 bis 30 $\mu$m.

Üblicherweise setzt man 6 bis 80, insbesondere 15 bis 65, bevorzugt 35 bis 50 Gew.-Teile Trägermaterial je 100 Gew.-Teile Ni ein.

Zur Herstellung homogener Copräzipitate ist es erforderlich, während der Fällung innerhalb eines bestimmten pH-Bereiches zu arbeiten. Im allgemeinen genügt es, einen pH-Bereich von 7 bis 10 einzuhalten. Will man besonders homogene Copräzipitate herstellen, so empfiehlt es sich, die Fällung in einem pH-Bereich von 7,3 bis 9, insbesondere 7,5 bis 8,5 vorzunehmen. Allzu große Schwankungen des pH-Wertes sollen während der Fällung vermieden werden. Man erreicht dies beispielsweise durch eine nicht zu schnelle, gleichmäßige Zugabe von Mischsalzlösung und Fällungsmittel.

Die Fällung erfolgt bei erhöhter Temperatur, die wenigstens 60 °C betragen sollte. Arbeitet man unter Druck, so können auch hohe Temperaturen angewendet werden. Im allgemeinen wird hierfür eine Temperatur von 120 °C angemessen sein. Eine Temperatur von 60 bis 120 °C ist ausreichend.

Gute Resultate liefern Fällungstemperaturen zwischen 70 bis 110, insbesondere zwischen 95 bis 105 °C. Die Temperatur während der Fällung beinflußt die Teilchengröße und die Schüttdichte. Sie soll daher möglichst konstant gehalten werden.

Es empfiehlt sich daher, die Temperatur während der Fällung entsprechend den gewünschten physikalischen Eigenschaften der Katalysatorzusammensetzung anzupassen. Da die Fällung nahezu quantitativ erfolgt, entspricht die Zusammensetzung des Fällungsgemisches den Mengenverhältnissen der Mischsalzlösung.

Nach Beendigung der Fällung wird, falls erforderlich auf Temperaturen von 70 °C und darunter abgekühlt, das Fällungsprodukt von der Mutterlauge abfiltriert, gewaschen, gegebenenfalls geformt, anschließend getrocknet und reduziert. Falls gewünscht kann die Katalysatorzusammensetzung anschließend z. B. durch gezielte Behandlung mit Luft oder Sauerstoff stabilisiert werden.

Durch Waschen mit Wasser werden unerwünschte, lösliche Bestandteile aus der gefällten Katalysatorzusammensetzung entfernt. Der Restgehalt der basischen Verbindung soll einer Menge unter 1, insbesondere unter 0,5, bevorzugt unter 0,2 Gew.-% $Na_2O$ entsprechen. Man wäscht daher solange, bis das Waschwasser diese Restgehalte aufweist.

Zur Formgebung der gewaschenen Katalysatorzusammensetzung kann man sich bewährter Verfahren, beispielsweise der Strangextrusion bedienen.

Die Trocknung wird bei erhöhten Temperaturen, vorzugsweise bei steigenden Temperaturen stufenweise durchgeführt. Die Trocknung in einem Temperaturbereich zwischen 40 bis 120, insbesondere 50 bis 100 °C bietet Gewähr, daß die gewünschte Restfeuchte (<10 Gew.-% Wasser bezogen auf Katalysatorzusammensetzung) eingestellt werden kann.

Die Reduktion erfolgt mittels Wasserstoff bei Temperaturen von 300 bis 550 °C, wobei ein Reduktionsgrad von wenigstens 80 %, insbesondere wenigstens 90 %, bevorzugt 95 % und darüber anzustreben ist.

Je höher der Reduktionsgrad liegt, desto reaktiver verhält sich die entsprechende Katalysatorzusammensetzung. Unter Reduktionsgrad versteht man Anteil Nickelmetall: Gesamtgehalt Nickel x 100 %.

Es ist bekannt, Nickel enthaltende Katalysatoren durch Zusatz von $ZrO_2$ einerseits und $Al_2O_3$ andererseits zu modifizieren. Jeder dieser beiden Promotoren wirkt sich günstig aus. Trotz guter Eignung derartiger trägerhaltiger Katalysatoren, die durch gemeinsame Fällung von Ni und $Al_2O_3$ oder Ni und $ZrO_2$ hergestellt werden können, besteht nach wie vor ein Bedarf nach Katalysatoren mit verbesserten Eigenschaften.

Es ist überraschend, daß diese Aufgabe gelöst wird durch eine Katalysatorzusammensetzung enthaltend Ni, $Al_2O_3$, und $ZrO_2$, dadurch gekennzeichnet, daß 20 bis 90, insbesondere 35 bis 75, bevorzugt 40 bis 70 Gew.-% Ni, bezogen auf Katalysatorzusammensetzung, sowie 1 bis 30 Gew.-Teile $Al_2O_3$ und 0,5 bis 20 Gew.-Teile $ZrO_2$ je 100 Gew.-Teile Ni als Copräzipitat auf einem Trägermaterial untergebracht sind.

Diese Katalysatorzusammensetzung ist den als Stand der Technik bekannten Katalysatoren überlegen. Die Herstellung erfolgt wie im vorangegangenen bereits beschrieben. Besonders günstig wirken sich feinteilige Trägermaterialien mit einer Korngröße von 0,1 bis 200, insbesondere 0,5 bis 50, bevorzugt 1,0 bis 30 μm aus. Als Trägermaterial können Aktivkohle, Tonerden, Bimsstein, $Al_2O_3$, $SiO_2$, Kieselgel, Kieselgur und Kieselerden verwendet werden. Besonders geeignet sind $SiO_2$, Kieselgel, Kieselgur und Kieselerde, insbesondere haben sich Kieselgur und $SiO_2$ in Form gefällter Kieselsäure bewährt.

Die guten Eigenschaften der Katalysatorzusammensetzung bleiben auch bei Veränderung der Gewichtsanteile der einzelnen Komponenten erhalten. Eine solche Katalysatorzusammensetzung besteht aus 35 bis 75 Gew.-% Nickel, bezogen auf Katalysatorzusammensetzung, 3 bis 15 Gew.-Teilen $Al_2O_3$ und 1 bis 10 Gew.-Teilen $ZrO_2$ je 100 Gew.-Teile Ni. Die Fällung von Ni, $Al_2O_3$ und $ZrO_2$ als Copräzipitat auf einen Träger verleiht der Katalysatorzusammensetzung die vorteilhafte Eignung.

Besonders kostengünstig lassen sich Katalysatorzusammensetzungen mit 40 bis 70 Ge.-% Ni, bezogen auf Katalysatorzusammensetzung, 4 bis 10 Gew.-Teilen $Al_2O_3$ und 1,5 bis 5 Gew.-Teilen $ZrO_2$ je 100 Gew.-Teile Ni herstellen. Trotz des enger begrenzten $Al_2O_3$ und $ZrO_2$-Anteiles besitzen diese Katalysatorzusammensetzungen Eigenschaften, die denen der zuvor aufgeführten Katalysatorzusammensetzungen entsprechen.

Die vorstehend beschriebenen Katalysatorzusammensetzungen lassen sich vorteilhaft bei der Hydrierung von Nitrilen, aromatischen Kohlenwasserstoffen, Nitroverbindungen und Olefinen in flüssiger Phase verwenden. Bereits bei mäßigen Temperaturen im Bereich von 50 bis 140 °C werden mit hoher Selektivität große Umsätze erzielt. Hierbei tritt die Überlegenheit der neuen Katalysatorzusammensetzung im Vergleich zu Ni und $Al_2O_3$ bzw. zu Ni und $ZrO_2$ haltigen Katalysatoren sehr deutlich hervor.

Darüber hinaus zeichnen sich die vorstehenden Katalysatorzusammensetzungen unter den bereits genannten Reaktionsbedingungen durch eine sehr hohe Beständigkeit aus.

Die nachfolgenden Beispiele dienen zur Verdeutlichung der vorliegenden Erfindung ohne sie zu begrenzen.

Beispiel 1

Herstellung einer Katalysatorzusammensetzung enthaltend 100 Gew.-Teile Ni, 6,5 Gew.-Teile $Al_2O_3$, 1,5 Gew.-Teile $ZrO_2$ und 45 Gew.-Teile Kieselgur.

305,76 g $Ni(NO_3)_2 \cdot 6H_2O$ und 29,52 g $Al(NO_3)_3 \cdot 9H_2O$ werden in 1760 ml destilliertem Wasser gelöst. Getrennt hiervon werden 2,32 g Zirkoncarbonat (Handelsware mit einem Zirkongehalt entsprechend 39,8 Gew.-% $ZrO_2$) in 9 ml technischer Salpetersäure (Gehalt 56 Gew.-% $HNO_3$) gelöst. Durch Vereinigung dieser beiden Lösungen erhält man die gewünschte Mischsalzlösung.

Das Fällungsmittel wird durch Auflösen von 147,04 g $Na_2CO_3$ in 1416 ml destilliertem Wasser hergestellt. Die Lösung enthält 104 g $NaCO_3$/l.

Man erhitzt die Mischsalzläsung auf 101°C und das Fällungsmittel auf 100°C und läßt die heiße Mischsalzlösung innerhalb von 3 Minuten gleichmäßig in die intensiv gerührte Sodalösung einlaufen.

In die frisch gefällte Suspension werden 27,76 g Kieselgur eingerührt und die entstehende Mischung wird noch weitere 3 Minuten gerührt.

Anschließend wird die Mutterlauge von dem Fällungsprodukt durch Filtration abgetrennt und das Fällungsprodukt mit 70°C heißem destillierten Wasser gewaschen, bis gegen Ende des Waschens der Alkaligehalt des Waschwassers 20 mg $Na_2O$/l beträgt.

Der Filterkuchen wird zur Weiterverarbeitung mit 70°C heißem, destilliertem Wasser aufgenommen (Mengenverhältnis Filterkuchen : destilliertem Wasser = 1 : 1) und suspendiert. Man rührt etwa 60 Minuten und filtriert erneut. Der danach anfallende Filterkuchen wird zu zylinderförmigen Formkörpern (Durchmesser 5 mm, Länge 8 bis 15 mm) extrudiert und anschließend bei steigender Temperatur (50 bis 75°C) mit Luft auf einen Restgehalt Wasser <10 Gew.-% Wasser, bezogen auf getrocknete Masse, getrocknet.

Das getrocknete Material wird im $H_2$-Strom (400 l $H_2$ je Liter Katalysator und Stunde) bei 470°C reduziert. Die Reduktion ist nach 4 Stunden beendet.

Die Katalysatorzusammensetzung enthält etwa 63 Gew.-% Ni und weist einen Reduktionsgrad von 99 % auf.

Durch vorsichtige Behandlung mit einem $O_2/N_2$-Gasgemisch (die Temperatur der Katalysatorzusammensetzung soll 100°C nicht übersteigen) kann der pyrophore Katalysator in eine stabilisierte, gegenüber Luft unempfindliche Form gebracht werden.

Beispiel 2

Herstellung einer Katalysatorzusammensetzung, enthaltend 100 Gew.-Teile Ni, 5 Gew.-Teile $Al_2O_3$, 3 Gew.-Teile $ZrO_2$ und 45 Gew.-Teile Kieselgur.

305,76 g $Ni(NO_3)_2 \cdot 6H_2O$ und 22,68 g $Al(NO_3)_3 \cdot 9H_2O$ werden in 1760 ml destilliertem Wasser gelöst. Getrennt hiervon werden 4,656 g Zirkoncarbonat (Handelsware mit einem Zirkongehalt entsprechend 39,8 Gew.-% $ZrO_2$) in 18 ml technische Salpetersäure (Gehalt 56 Gew.-% $HNO_3$) gelöst. Durch Vereinigung dieser beiden Lösungen erhält man die gewünschte Mischsalzlösung.

Das Fällungsmittel wird durch Auflösen von 175,2 g $Na_2CO_3$ in 1680 ml destilliertem Wasser hergestellt. Die Lösung enthält 104 g $Na_2CO_3$/l.

Anschließend verfährt man wie in Beispiel 1 angegeben, erhitzt die Mischsalzlösung auf 101°C und das Fällungsmittel auf 100°C und läßt die heiße Mischsalzlösung innerhalb von 3 Minuten gleichmäßig in die intensiv gerührte Sodalösung einlaufen. In die frisch gefällte Suspension werden 27,76 g Kieselgur eingerührt und die entstehende Mischung wird noch weitere 3 Minuten gerührt.

Anschließend wird wie in Beispiel 1 angegeben filtriert, gewaschen, mit destilliertem Wasser suspendiert, erneut filtriert, extrudiert, getrocknet, mit $H_2$ reduziert und gegebenenfalls mit einem $O_2/N_2$- Gasgemisch stabilisiert.

Beispiele 3a bis 3c

Hydrierung von Nitrobenzol.

394 g Nitrobenzol, 150 g Wasser und 0,95 g Katalysator werden in einem 1 l-Autoklaven unter Rühren bei 30 bar $H_2$-Druck und 130°C zur Umsetzung gebracht. Die Ergebnisse unter Verwendung verschiedener Katalysatoren sind der nachfolgenden Tabelle 1 zu entnehmen.

Tabelle 1

| Beispiel | 3a | 3b (Vergleich) | 3c (Vergleich) |
|---|---|---|---|
| Katalysator[1] : | | | |
| Ni | 100 | 100 | 100 |
| $Al_2O_3$ | 5 | 8 | - |
| $ZrO_2$ | 3 | - | 8 |
| $SiO_2$ | 45 | 45 | 45 |
| Reaktionszeit (min)[2] | 90 | 115 | 180 |
| Reaktionsprodukt[3] : | | | |
| Cyclohexylamin | 0,17 | 1,3 | 0,8 |
| Anilin | 99,8 | 97,6 | 95,3 |
| Nebenprodukte[4] | 0,03 | 2,1 | 3,9 |
| Nitrobenzol | - | - | 0,2 |

1) Angaben in Gew.-Teilen, hergestellt wie unter Beispiel 2 beschrieben
2) Ende der $H_2$-Aufnahme
3) Angaben in Gew.-% ermittelt durch gaschromatografische Analyse
4) unter anderem Azobenzol, Azoxybenzol und Hydrazobenzol

Beispiele 4a bis 4c

Hydrierung von aromatischen Kohlenwasserstoffen.

400 g eines Kohlenwasserstoffgemisches (Handelsprodukt Esso Varsol: Siedebereich 140 bis 170°C, Aromatengehalt 24,3 Gew.-%) werden mit 3,3 g Katalyator in einem 1 l-Autoklaven unter Rühren bei 20 bar $H_2$-Druck und 140°C zur Umsetzung gebracht.

Die Ergebnisse unter Verwendung verschiedener Katalysatoren sind der nachfolgenden Tabelle 2 zu entnehmen.

Tabelle 2

| Beispiel | 4a | 4b (Vergleich) | 4c (Vergleich) |
|---|---|---|---|
| Katalysator[1] | I | II | III |
| Reaktionszeit (min) | 100 | 125 | 195 |
| Aromatenanteil | < 1 ppm | 3 ppm | 37 ppm |

1) Vergleiche Angaben in Tabelle 1

Beispiele 5a und 5b

Hydrierung von Nitrilen zu primären Aminen.

300 g Talgfettsäurenitril (Jodzahl 51) werden mit Katalysator und 17,5 g $NH_3$ versetzt und in einem 1 l-Autoklaven unter Rühren bei 30 bar ($H_2$ + $NH_3$)-Druck und 135°C umgesetzt. In Beispiel 5a werden 1,0 Katalysator I und in Beispiel 5b (Vergleich) 2,5 g Katalysator II eingesetzt. Die Ergebnisse der Versuche sind Tabelle 3 zu entnehmen.

Tabelle 3

| Beispiel | 5a | 5b (Vergleich) |
|---|---|---|
| Katalysator[1] | I | II |
| Reaktionszeit (min)[2] | 150 | 145 |
| Reaktionsprodukt[3] | | |
| primäres Amin | 95,5 | 90,5 |
| sek. und tert. Amin | 4,3 | 8,5 |
| Jodzahl | 49 | 50 |

1) Vergleiche Angaben in Tabelle 1
2) Ende der $H_2$-Aufnahme
3) Angaben in Gew.-% ermittelt durch gaschromatografische Analyse

**Patentansprüche**

1.  Verfahren zur Herstellung einer Nickel, Aluminiumoxid und Zirkondioxid enthaltenden Katalysatorzusammensetzung durch Fällung, Filtration, Trocknung und Reduktion, dadurch gekennzeichnet, daß eine wäßrige Ni-Al-Zr-Mischsalzlösung mit einer wäßrigen Lösung einer basischen Verbindung als Fällungsmittel vermischt, die basische Verbindung in einem stöchiometrischen Überschuß von 5 bis 100 %, bezogen auf die zur Fällung von Ni, Al und Zr erforderliche Menge, eingesetzt wird, Ni, Al und Zr gleichzeitig gefällt und auf einem Trägermaterial abgeschieden werden, die Fällung bei 60 bis 120°C und pH 7 bis 10 durchgeführt wird.

2.  Verfahren nach Anspruch 1, dadurch gekennzeichnet, daß die Mischsalzlösung freie Säure entsprechend einem Verhältnis $H^+ : Zr^{4+} = (2 \text{ bis } 40) : 1$ enthält.

3.  Verfahren nach Anspruch 1 oder 2, dadurch gekennzeichnet, daß als freie Säure Salpetersäure eingesetzt wird.

4.  Verfahren nach einem oder mehreren der Ansprüche 1 bis 3, dadurch gekennzeichnet, daß die Mischsalzlösung 10 bis 100 g Ni/l enthält.

5.  Verfahren nach einem oder mehreren der Ansprüche 1 bis 4, dadurch gekennzeichnet, daß die Mischsalzlösung Aluminium entsprechend 1 bis 30, insbesondere 3 bis 15, bevorzugt 4 bis 10 Gew.-Teilen $Al_2O_3$ je 100 Gew.-Teile Ni enthält.

6.  Verfahren nach einem oder mehreren der Ansprüche 1 bis 5, dadurch gekennzeichnet, daß die Mischsalzlösung Zirkon entsprechend 0,5 bis 20, insbesondere 1 bis 10, bevorzugt 1,5 bis 5 Gew.-Teilen $ZrO_2$ je 100 Gew.-Teile Ni enthält.

7.  Verfahren nach einem oder mehreren der Ansprüche 1 bis 6, dadurch gekennzeichnet, daß Nickel, Zirkon und Aluminium in Form ihrer Sulfate, Chloride, Acetate, Propionate, Butyrate und Nitrate, insbesondere in Form ihrer Nitrate eingesetzt werden.

8.  Verfahren nach einem oder mehreren der Ansprüche 1 bis 7, dadurch gekennzeichnet, daß als Fällungsmittel eine wäßrige Alkalicarbonat-, Alkalihydrogencarbonat-, Alkalihydroxid-, Ammoniumhydroxid- und/oder Ammoniumcarbonatlösung eingesetzt wird.

9.  Verfahren nach einem oder mehreren der Ansprüche 1 bis 8, dadurch gekennzeichnet, daß das Fällungsmittel vorgelegt wird und die Mischsalzlösung in das Fällungsmittel eingeleitet wird.

10. Verfahren nach einem oder mehreren der Ansprüche 1 bis 9, dadurch gekennzeichnet, daß die Mischsalzlösung und das Fällungsmittel miteinander vermischt werden und anschließend das Trägermaterial zugesetzt wird.

**11.** Verfahren nach einem oder mehreren der Ansprüche 1 bis 10, dadurch gekennzeichnet, daß das Trägermaterial eine Korngröße von 0,1 bis 200, insbesondere 0,5 bis 50, bevorzugt 1 bis 30 $\mu$m aufweist.

**12.** Verfahren nach einem oder mehreren der Ansprüche 1 bis 11, dadurch gekennzeichnet, daß die Fällung bei pH 7,3 bis 9,0, insbesondere 7,5 bis 8,5 durchgeführt wird.

**13.** Verfahren nach einem oder mehreren der Ansprüche 1 bis 12, dadurch gekennzeichnet, daß die Fällung bei 70 bis 110 °C, insbesondere 95 bis 105 °C durchgeführt wird.

**14.** Katalysatorzusammensetzung enthaltend Ni, $Al_2O_3$ und $ZrO_2$, dadurch gekennzeichnet, daß 20 bis 90 Gew.-% Ni bezogen auf Katalysatorzusammensetzung, sowie 1 bis 30 Gew.-Teile $Al_2O_3$ und 0,5 bis 20 Gew.-Teile $ZrO_2$ je 100 Gew.-Teile Ni als Copräzipitat auf einem Trägermaterial untergebracht sind.

**15.** Katalysatorzusammensetzung nach Anspruch 14, dadurch gekennzeichnet, daß sie 35 bis 75 Gew.-% Ni, bezogen auf Katalysatorzusammensetzung, 3 bis 15 Gew.-Teile $Al_2O_3$ und 1 bis 10 Gew.-Teile $ZrO_2$ je 100 Gew.-Teile Ni enthält.

**16.** Katalysatorzusammensetzung nach Anspruch 14, dadurch gekennzeichnet, daß sie 40 bis 70 Gew.-% Ni, bezogen auf Katalysatorzusammensetzung, 4 bis 10 Gew.-Teile $Al_2O_3$ und 1,5 bis 5 Gew.-Teile $ZrO_2$ je 100 Gew.-Teile Ni enthält.

**17.** Verwendung der Katalysatorzusammensetzung gemäß Anspruch 14 als hochaktiver Katalysator für Hydrierungen von Nitrilen, aromatischen Kohlenwasserstoffen, Nitroverbindungen und Olefinen in flüssiger Phase.

**Claims**

**1.** A process for preparing a catalyst composition containing nickel, aluminium oxide and zirconium dioxide by precipitation, filtration, drying and reduction, characterised in that an aqueous Ni-Al-Zr mixed-salt solution is mixed with an aqueous solution of a basic compound as a precipitant, the basic compound is used in a stoichiometric excess of 5 to 100 %, related to the amount required for the precipitation of Ni, Al and Zr, the nickel, aluminium and zirconium are simultaneously precipitated and deposited onto a support material, the precipitation takes place at 60 to 120°C and a pH value of 7 to 10.

**2.** A process according to claim 1, characterised in that the mixed-salt solution contains free acid corresponding to a ratio of $H^+ : Zr^{4+} = (2 \text{ to } 40) : 1$.

**3.** A process according to claim 1 or 2, characterised in that nitric acid is used as a free acid.

**4.** A process according to one or more of the claims 1 to 3, characterised in that the mixed-salt solution contains 10 to 100 g of Ni/l.

**5.** A process according to one or more of the claims 1 to 4, characterised in that the mixed-salt solution contains aluminium corresponding to 1 to 30, in particular 3 to 15, preferably 4 to 10 parts by weight of $Al_2O_3$ per 100 parts by weight of nickel.

**6.** A process according to one or more of the claims 1 to 5, characterised in that the mixed-salt solution contains zirconium corresponding to 0.5 to 20, in particular 1 to 10, preferably 1.5 to 5 parts by weight of $ZrO_2$ per 100 parts by weight of nickel.

**7.** A process according to one or more of the claims 1 to 6, characterised in that nickel, zirconium and aluminium are used in the form of their sulfates, chlorides, acetates, propionates, butyrates and nitrates, in particular in the form of their nitrates.

**8.** A process according to one or more of the claims 1 to 7, characterised in that an aqueous alkali metal carbonate, alkali metal hydrogen carbonate, alkali metal hydroxide, ammonium hydroxide and/or ammonium carbonate solution is used as the precipitant.

9. A process according to one or more of the claims 1 to 8, characterised in that the precipitant is taken and the mixed-salt solution is introduced into the precipitant.

10. A process according to one or more of the claims 1 to 9, characterised in that the mixed-salt solution and the precipitant are mixed with each other and the support material is then added.

11. A process according to one or more of the claims 1 to 10, characterised in that the support material exhibits a particle size of 0.1 to 200, in particular 0.5 to 50, preferably 1 to 30 $\mu$m.

12. A process according to one or more of the claims 1 to 11, characterised in that precipitation is performed at a pH value of 7.3 to 9.0, in particular 7.5 to 8.5.

13. A process according to one or more of the claims 1 to 12, characterised in that precipitation is performed at 70 to 110°C, in particular 95 to 105°C.

14. Catalyst composition containing Ni, $Al_2O_3$ and $ZrO_2$, characterised in that 20 to 90 wt.% of nickel, related to the catalyst composition, as well as 1 to 30 parts by weight of $Al_2O_3$ and 0.5 to 20 parts by weight of $ZrO_2$, in each case per 100 parts by weight of nickel, as coprecipitate are located on a support material.

15. A catalyst composition according to claim 14, characterised in that it contains 35 to 75 wt.% of Ni, related to the catalyst composition, 3 to 15 parts by weight of $Al_2O_3$ and 1 to 10 parts by weight of $ZrO_2$, in each case per 100 parts by weight of nickel.

16. A catalyst composition according to claim 14, characterised in that it contains 40 to 70 wt.% of Ni, related to the catalyst composition, 4 to 10 parts by weight of $Al_2O_3$ and 1.5 to 5 parts by weight of $ZrO_2$, in each case per 100 parts by weight of nickel.

17. Use of the catalyst composition according to claim 14 as a highly active catalyst for hydrogenating nitriles, aromatic hydrocarbons, nitro-compounds and olefins in the liquid phase.

**Revendications**

1. Procédé pour la fabrication d'une composition de catalyseur contenant du nickel, de l'oxyde 'd'aluminium et du dioxyde de zirconium par précipitation, filtration, lavage et réduction, caractérisé en ce que l'on mélange une solution saline mixte de sels de Ni, Al, Zr avec une solution aqueuse d'un composé basique comme agent précipitant, on utilise le composé basique en excès stoechiométrique de 5 à 100% par rapport à la quantité nécessaire pour la précipitation de Ni, Al et Zr, on précipite simultanément Ni, Al et Zr et on les dépose sur une matière de support, la précipitation étant mise en oeuvre à 60-120°C et à pH 7-10.

2. Procédé selon la revendication 1, caractérisé en ce que la solution saline mixte contient un acide libre en quantité correspondant à un rapport $H^+/Zr^{4+}$ de (2 à 40):1.

3. Procédé selon la revendication 1 ou 2, caractérisé en ce que l'on utilise comme acide libre l'acide nitrique.

4. Procédé selon une ou plusieurs des revendications 1 à 3, caractérisé en ce que la solution saline mixte contient 10 à 100 g de Ni/l.

5. Procédé selon une ou plusieurs des revendications 1 à 4, caractérisé en ce que la solution saline mixte contient de l'aluminium en quantité correspondant à 1 à 30 parties en poids d'$Al_2O_3$, en particulier 3 à 15, de préférence 4 à 10 parties en poids, par 100 parties en poids de Ni.

6. Procédé selon une ou plusieurs des revendications 1 à 5, caractérisé en ce que la solution saline mixte contient du zirconium en quantité correspondant à 0,5 à 20 parties en poids de $ZrO_2$ par 100 parties en poids de Ni, en particulier 1 à 10, de préférence 1,5 à 5 parties en poids pour 100 parties en poids.

7. Procédé selon ou plusieurs des revendications 1 à 6, caractérisé en ce que le nickel, le zirconium et l'aluminium sont mis en jeu sous forme de leurs sulfates, chlorures, acétates, propionates, butyrates et nitrates, en particulier sous forme de leurs nitrates.

8. Procédé selon ou plusieurs des revendications 1 à 7, caractérisé en ce que l'on utilise comme agent précipitant une solution aqueuse de carbonate alcalin, de bicarbonate alcalin, d'hydroxyde alcalin, d'hydroxyde d'ammonium et/ou de carbonate d'ammonium.

9. Procédé selon une ou plusieurs des revendications 1 à 8, caractérisé en ce que l'on charge au préalable l'agent précipitant et on introduit la solution saline mixte dans l'agent précipitant.

10. Procédé selon une ou plusieurs des revendications 1 à 9, caractérisé en ce que la solution saline mixte et l'agent précipitant sont mélangés l'une avec l'autre et on ajoute ensuite la matière de support.

11. Procédé selon une ou plusieurs des revendications 1 à 10, caractérisé en ce que la matière de support présente une grosseur de grains de 0,1 à 200 $\mu$m, en particulier de 0,5 à 50, de préférence 1 à 30 $\mu$m.

12. Procédé selon une ou plusieurs des revendications 1 à 11, caractérisé en ce que la précipitation est mise en oeuvre à un pH de 7,3 à 9,0, en particulier 7,5 à 8,5.

13. Procédé selon une ou plusieurs des revendications 1 à 12, caractérisé en ce que la précipitation est mise en oeuvre à 70-110°C, en particulier 95 à 105°C.

14. Composition de catalyseur contenant Ni, $Al_2O_3$ et $ZrO_2$, caractérisée en ce que l'on applique sous forme de coprécipité sur une matière de support 20 à 90% en poids de Ni, par rapport à la composition de catalyseur, et 1 à 30 parties en poids d'$Al_2O_3$ et 0,5 à 20 parties en poids de $ZrO_2$ par 100 parties en poids de Ni.

15. Composition de catalyseur selon la revendication 14, caractérisée en ce qu'elle contient 35 à 75% en poids de Ni, par rapport à la composition de catalyseur, 3 à 15 parties en poids d'$Al_2O_3$ et 1 à 10 parties en poids de $ZrO_2$ par 100 parties en poids de Ni.

16. Composition de catalyseur selon la revendication 14, caractérisée en ce qu'elle contient 40 à 70% en poids de Ni, par rapport à la composition d'un catalyseur, 4 à 10 parties en poids d'$Al_2O_3$ et 1,5 à 5 parties en poids de $ZrO_2$ par 100 parties en poids de Ni.

17. Utilisation de la composition de catalyseur selon la revendication 14 comme catalyseur d'activité élevée pour l'hydrogénation en phase liquide de nitriles, d'hydrocarbures aromatiques, de composés nitrés et d'oléfines.